# EUROPÄISCHE PATENTSCHRIFT

(11) **EP 2 486 954 B1**
(45) Veröffentlichungstag und Bekanntmachung des Hinweises auf die Patenterteilung: **14.10.2015**
(21) Anmeldenummer: 12151781.7
(22) Anmeldetag: 19.01.2012
(51) Int. Cl.: A61N 1/375, H01G 9/10, H01G 9/008

(54) **Kontaktdurchführung bei elektronischen Komponenten**
Feedthrough conductor for electronic components
Passage de contact dans des composants électroniques

(30) Priorität: 10.02.2011 US 201161441287 P
(43) Veröffentlichungstag der Anmeldung: 15.08.2012
(73) Patentinhaber: LITRONIK Batterietechnologie GmbH, 01796 Pirna (DE)
(72) Erfinder: Pretzlaff, Bernd, 25866 Milstedt (DE); Lippitz, Holger, 12205 Berlin (DE)
(74) Vertreter: Galander, Marcus

(56) Entgegenhaltungen:
- EP-A2- 0 083 271
- WO-A1-2010/028433
- WO-A2-02/43090
- DE-A1- 1 564 766
- DE-A1- 2 645 323
- US-A- 3 354 359
- US-A- 4 296 458
- US-A1- 2008 043 403
- US-A1- 2008 190 647
- US-A1- 2008 259 528
- US-B1- 7 696 002
- US-B1- 7 837 085

## Beschreibung

Die Erfindung befasst sich mit elektrischen Durchführungen, insbesondere mit elektrischen Durchführungen für Bauteile in elektrischen Implantaten, die in den menschlichen Körper eingesetzt werden.

Elektronische Implantate haben als Mikrostimulatoren in vielfältiger Ausführungsform Anwendung in der modernen Medizintechnik gefunden und werden beispielsweise als Herzschrittmacher, Defibrillatoren (ICD's, CRT-D's) oder Neurostimulatoren eingesetzt.

Derartige Implantate weisen im allgemeinen eine Anzahl elektrischer Kontakte auf, die einerseits zur Aufnahme und Weiterleitung von mittels Sonden gemessener elektrischer Impulse dienen, andererseits sind auch Elektrodenleitungen vorgesehen, die Reizsignale von einer Steuereinheit zur Stimulation an eine oder mehrere implantierte Elektroden weiterleiten. So kann beispielsweise ein Herzschrittmacher die vom Sinusknoten generierten Anregungsimpulse des Herzschlags detektieren und bei Störungen z.B. der Schlagfolge stimulierende elektrische Signale an eine oder mehrere Reizelektroden am Herz abgeben.

Eine derartige implantierbare Anordnung zur Stimulation ist beispielsweise beschrieben in der US 2008/0154320 A1. Der in dieser Schrift dargestellte Herzschrittmacher besteht aus einem Gehäuse, in dem neben einer Batterie und der biometrischen Auswerte- und Steuerelektronik insbesondere auch ein Kondensator untergebracht ist, der zur Generierung und Übertragung elektrischer Reizimpulse an mindestens eine aus dem Gehäuse herausgeführte Elektrodenleitung dient.

Insbesondere bei Anregungssignalen, die direkt auf zentrale Funktionen menschlicher Organe einwirken, sind die Anforderungen an die Qualität der übertragenen Signale selbstverständlich sehr hoch. Dies betrifft einerseits die präzise zeitliche Gabe eines Anregungsimpulses oder einer Impulsfolge, andererseits aber auch die Regulierung und Stabilisierung der geeigneten Pulsform und der elektrischen Intensität.

Die Eigenschaften des in das Implantat integrierten Kondensators bestimmen dabei zu einem nicht unwesentlichen Teil die erreichbare Intensität und Güte der übertragenen Signalform. Vorgeschlagen wird daher in der US 2008/0154320 A1 die Verwendung von speziellen Oxiden zur Bildung einer Trennschicht zwischen Anode und Kathode des Kondensators, die aufgrund hoher Werte der Dielektrizitätskonstanten eine möglichst hohe Kapazität bei gleichzeitig möglichst kleinem Kondensatorvolumen bereitstellt.

Implantierte Stimulatoren können heute zum Teil über Jahre im menschlichen Körper verbleiben. Das bedingt, dass sowohl das Gehäuse als auch die Elektrodenleitungen eines solchen Implantats aus einem Material bestehen, das eine möglichst hohe Körperverträglichkeit (Biokompatibilität) aufweist, so dass keine Stoffe in den Körper abgegeben werden, die den Metabolismus schädigen könnten.

Jedes eingesetzte Implantat ist dabei über den gesamten Funktionszeitraum von Körpermedium umgeben, eine Mischung verschiedener chemischer Bestandteile, die insbesondere aufgrund der unterschiedlichen gelösten Ionen relativ reaktionsfähig ist. Dies stellt besondere Anforderungen an eine dauerhafte hermetische Abdichtung des Implantatgehäuses. Dabei gilt es einerseits, ein Eindringen von Körperflüssigkeit zu verhindern und damit den Schutz aller inneren elektronischen Komponenten zu gewährleisten, als auch andererseits zu verhindern, dass ein Austritt intern vorhandener und unter Umständen auf den Organismus schädigend wirkende Substanzen (Batteriemedien, Kondensatorelektrolyte) ins Körpermedium erfolgen kann.

Die Abdichtung im Bereich einer Durchführung einer Elektrode durch eine Öffnung in der Gehäusewandung des Implantats ist dabei besonders problematisch. Um in diesem kritischen Bereich eine zuverlässige Trennung von externem Körpermedium und beispielsweise intern im Kondensator befindlichem Elektrolyt zu erreichen, muss die Abdichtung aus alterungsbeständigem Material bestehen, das entsprechende mechanische Eigenschaften für eine Versiegelung aufweist und stabil gegenüber den chemischen Einwirkungen von Körperflüssigkeit und den unter Umständen intern angrenzenden Substanzen ist. Kontaktdurchführungen von Kondensatoren werden unter anderem in der EP0083271A2, US7696002B1. US2008/190647, US2008/043403, DE2645323A1 und US3354359A beschrieben.

Des Weiteren werden auch hohe Anforderungen an die Durchführungen der im Implantat verwendeten elektronischen Komponenten gestellt, insbesondere an Batterien oder Kondensatoren. So sollen beispielsweise die elektrischen Durchführungen eines Kondensators für den Elektrolyten eine, zumindest während der zu erwartenden Lebensdauer, unüberwindbare Barriere darstellen, um das Innere des Implantats vor austretender Elektrolytlösung zu schützen und die Funktionsfähigkeit des Kondensators und damit des Implantats nicht zu gefährden.

Die metallische Elektrode der Durchführung wird dazu üblicherweise im Bereich des Durchtritts von elastischem Dichtungsmaterial umgeben. Geeignet sind zum Beispiel Kunststoffe auf Elastomer-Basis, wobei im Falle eines elektrisch leitfähigem Gehäusematerials das Dichtungsmaterial gleichzeitig auch als elektrische Isolierung dient. Dabei müssen im Herstellungsprozess für eine dauerhafte hermetische Versiegelung des Elektrodendurchgangs alle aneinander grenzenden Teilflächen, also zwischen Elektrode und Dichtungsmaterial und ebenso zwischen Dichtungsmaterial und Gehäusewandung, fugen- und porenfrei aneinander gefügt werden.

Die Schrift US 7,365,960 liegt auf diesem technischen Gebiet. Beschrieben wird darin eine Elektrodendurchführung eines Kondensators für Implantate mit einer Abdichtung mittels eines speziell ausgeformten Dichtungsflansches.

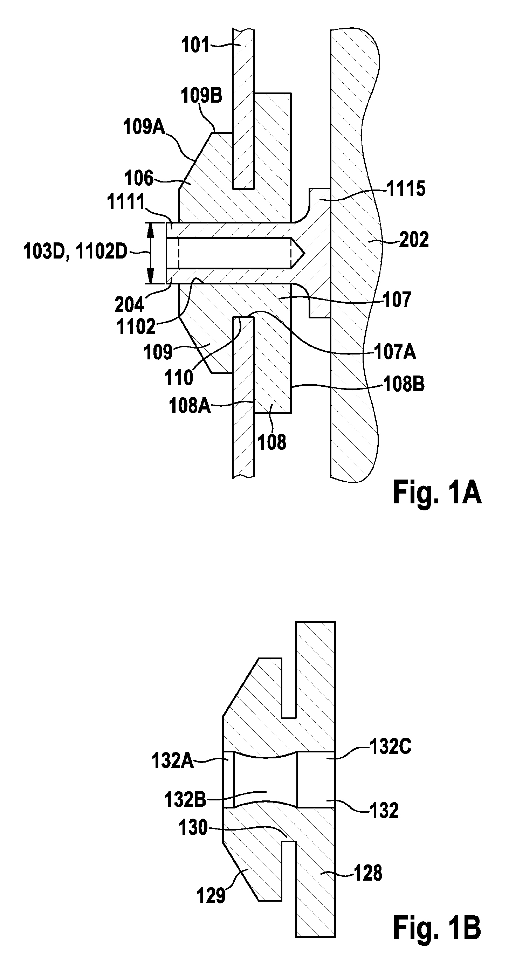

Die Figuren 1a und 1b (Stand der Technik) zeigen einen Querschnitt der Elektrodendurchführung und des darin eingesetzten Dichtungsflansches nach der US 7,365,960. Der aus Gummi oder elastischem Kunststoff bestehende Dichtungsflansch besitzt eine innere Durchgangsöffnung, durch die ein leitfähiges Verbindungsstück vom im Implantat befindlichen Kondensator hindurchgeführt wird. Das Verbindungsstück ist dabei als Hohlzylinder ausgeführt, in den die weiterführende Elektrodenleitung von außen zentral eingesteckt ist. Da die Durchgangsöffnung des Dichtungsflansches einen kleinen Querschnitt als der Elektrodendurchmesser aufweist (vergl. FIG. 1B), entsteht beim Zusammensetzen der Anordnung ein entsprechender Andruck, wodurch die Abdichtung zwischen Elektrode und Flansch gewährleistet wird. Für die Abdichtung gegenüber dem Gehäuse weist der Flansch eine äußere, ringförmig umlaufende Nut auf, in die nach dem Einsetzen des Flansches die Gehäusewandung eingreift. Auch die Maße der Nut sind etwas kleiner als die Stärke der Gehäusewandung vorgesehen, so dass nach Einsetzen des Flansches in die Gehäuseöffnung das elastische Dichtungsmaterial an der Gehäusewandung beidseitig dicht anliegt.

Die vorgeschlagene Anordnung zur Durchführung eines Kondensatorkontakts bei Implantaten hat den Nachteil, dass aufgrund der speziellen Formgebung des Dichtungsflansches der Fertigungsaufwand relativ aufwändig ist, da beim Zusammenfügen der Anordnung die umlaufende Nut des Flansches präzise in die Gehäuseöffnung eingepasst werden muss. Auch die elektrische Durchführung mittels Steckverbindung zwischen weiterführender äußerer Elektrodenleitung und metallischem Verbindungsstück zum Kondensator erfordert die möglichst passgenaue Anfertigung und entsprechendes Ineinanderfügen dieser Übergangsstücke.

Die vorliegende Erfindung geht aus von der in der US 7,365,960 offenbarten Kontaktdurchführung für Implantate als nächstliegendem Stand der Technik. Ihr liegt die Aufgabe zugrunde, für elektronische Komponenten, wie etwa in dauerimplantierbare Stimulatoren, eine Kontaktdurchführung zu entwickeln, die die Anforderungen an Versiegelung erfüllt und zusätzliche Vorteile aufweist, wobei ein einfaches Zusammenfügen von Elektrode, Dichtungsmaterial und Gehäuse ermöglicht ist. Eine weitere Aufgabe der Erfindung ist die Bereitstellung einer derartigen Kontaktdurchführung für Kondensatoren, bei der zusätzlich eine Formierung bzw. Anodisierung der Elektrodenoberfläche des Kondensatorkontakts bis zu einem definierten Bereich der Durchführung erfolgen kann.

Diese Aufgabe wird bei einer Kontaktdurchführung für dauerimplantierbare Stimulatoren mit den Merkmalen des Oberbegriffs des Anspruchs 1 gelöst durch die kennzeichnenden Merkmale des Anspruchs 1. Weitere Merkmale vorteilhafter Ausführungsformen der Erfindung sind Gegenstand der Unteransprüche.

Erfindungsgemäß weist eine metallische Elektrode im Bereich der Durchführung durch eine Öffnung im Komponentengehäuse einen lokal vergrößerten Durchmesser auf, der so ausgelegt ist, dass ein schlauchförmig um die Elektrode angeordnetes elastisches Dichtungsmaterial in diesem Bereich aufgeweitet wird. Damit liegt dort das elastische Dichtungsmaterial unter entsprechendem Druck auf der Elektrode eng an, was eine zuverlässige Abdichtung zwischen Elektrodenoberfläche und Dichtungsmaterial bewirkt. Durch die Aufweitung wird das schlauchförmige Dichtungsmaterial auch an die Wandung der Öffnung im Komponentengehäuse angepresst, wodurch in diesem Bereich die erforderliche Abdichtung zur Gehäusewandung gewährleistet ist. Zur Fixierung der Elektrode in dieser Position ist auf der von der Komponente wegführenden Seite der Durchführung ein stark haftendes Material aufgebracht, z. B. Klebstoff, Kunstharz o. ä., das einerseits die Elektrode umschließt und andererseits die Öffnung im Komponentengehäuse vollständig überdeckt. Damit ist neben der Fixierung der Elektrode und des Dichtungsmaterials in der gewünschten Position auch eine zusätzliche Versiegelung der Elektrodendurchführung gegenüber dem Außenraum gewährleistet. Außerdem ist vorgesehen, dass der von der Abdichtung aus ins Innere der elektronischen Komponente führende Abschnitt der Elektrode als Kondensatorkontakt ausgeführt ist und
- in diesem Bereich vom Dichtungsmaterial schlauchförmig umgeben ist, wobei dieser Abschnitt der Elektrode eine Oberflächenstruktur in Form von Vertiefungen und/oder Erhebungen dergestalt aufweist, dass zwischen Dichtungsmaterial und Elektrodenoberfläche Kapillaren verbleiben, über die Flüssigkeiten zur Elektrodenoberfläche diffundieren kann, oder
- vom Dichtungsmaterial schlauchförmig umgeben ist und wobei das Dichtungsmaterial nur in diesem Bereich zur Elektrodeseite hin eine Oberflächenstruktur in Form von Vertiefungen und/oder Erhebungen dergestalt aufweist, dass zwischen Dichtungsmaterial und Elektrodenoberfläche Kapillaren verbleiben, über die Flüssigkeiten zur Elektrodenoberfläche diffundieren kann.

Die Erfindung wird im Folgenden anhand bevorzugter Ausführungsbeispiele unter Bezugnahme auf die Zeichnungen und die darin aufgeführten Bezugszeichen näher beschrieben.

Es zeigen:
- FIG. 1A, 1B:: Stand der Technik, Kontaktdurchführung gemäß US 7,365,960
- FIG. 2:: Schnitt durch ein erstes Ausführungsbeispiel der erfindungsgemäßen Kontaktdurchführung
- FIG 3:: ein Ausführungsbeispiel für eine erfindungsgemäße riefenförmige Strukturierung der Oberfläche eines Elektrodenabschnitts zur Anodisierung
- FIG. 4:: ein Ausführungsbeispiel für eine erfindungsgemäße stegförmige Strukturierung der Oberfläche eines Elektrodenabschnitts
- FIG 5:: ein Ausführungsbeispiel für eine alternative geometrische Anordnung einer erfindungsgemäßen Strukturierung der Oberfläche eines Elektrodenabschnitts.
- FIG 6:: Ausführungsbeispiel für eine erfindungsgemäße Strukturierung der Innenfläche des Dichtungsmaterials
- FIG 7:: Ausführungsbeispiel für eine erfindungsgemäße partielle Perforierung des Dichtungsmaterials

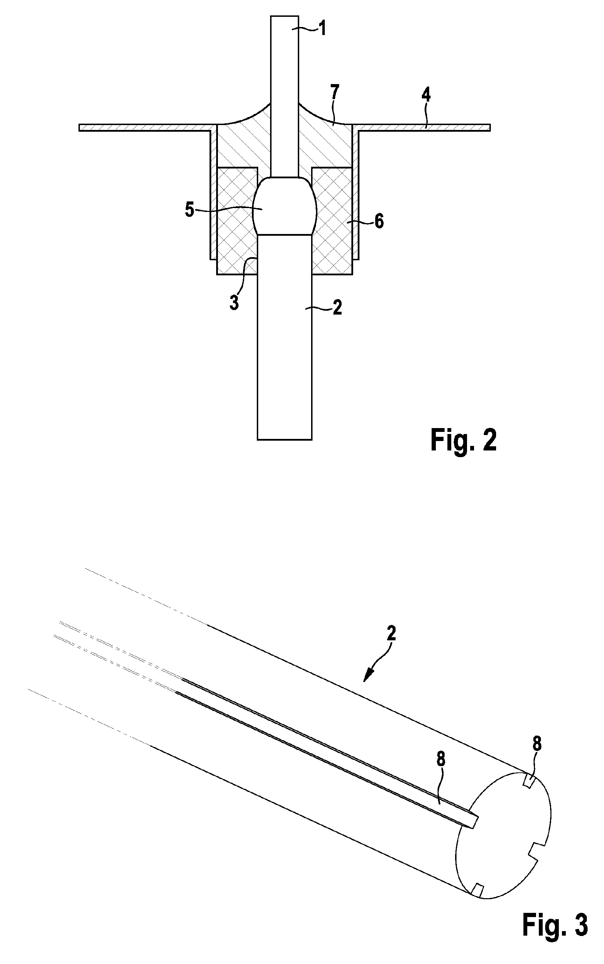

In der Figur 2 ist eine erste Ausführungsform der erfindungsgemäßen Kontaktdurchführung als Schnittbild dargestellt. In diesem Beispiel besteht der durchgeführte Kontakt aus zwei Abschnitten: einem von einer Komponente, wie beispielsweise einem Kondensator oder einer Batterie, wegführenden äußeren Abschnitt 1 und einem inneren Abschnitt 2. Im Bereich der Öffnung 3 in der Wandung 4 der Komponente ist der Querschnitt der Elektrode 2 lokal aufgeweitet. In diesem Ausführungsbeispiel ist die Aufweitung als Rundprofil geformt. Der so gebildete Wulst 5 übt auf das schlauchförmig um die Elektrode 2 angeordnete elastische Dichtungsmaterial 6 Druck aus, so dass eine Abdichtungswirkung zwischen Elektrodenoberfläche und Dichtungsmaterial einerseits und Dichtungsmaterial und Wandung der Gehäuseöffnung 3 andererseits bewirkt wird. Auf der Außenseite der Komponente ist die Kontaktdurchführung mittels eines adhäsiven Materials 7, z. B. Epoxydharz, versiegelt. Dabei kann dieses Material vorzugsweise auch zur mechanischen Fixierung der Elektrode 1 in der dargestellten Position dienen, so dass die Kontaktdurchführung gegen eine eventuelle Schuboder Zugbelastung der äußeren Elektrode 1 stabilisiert ist.

Bei der Anfertigung der Elektrodenanordnung ist eine korrekte Positionierung der Elektrode 2 zusammen mit dem schlauchförmigen Dichtungsmaterial 6 innerhalb der Öffnung 3 wesentlich. Fertigungstechnisch ist es dazu vorteilhaft, wenn die Öffnung 3 lokal, vorzugsweise im zentralen Bereich des Durchgangs, einen etwas vergrößerten Querschnitt aufweist (in Fig. 2 nicht dargestellt). Damit greift dann nach entsprechender Montage der Elektrodenabschnitt 2 über das im Bereich der wulstförmigen Verdickung 5 aufgeweitete Dichtungsmaterial 6 formschlüssig in diesen Bereich der Öffnung ein. Auf diese Weise kann beispielsweise die Elektrode 2 zusammen mit dem aufgeschobenen Dichtungsschlauch 6 einfach bis zum passgenauen Sitz der wulstförmigen Aufweitung 5 in die Öffnung 3 eingeschoben werden. Die Dimensionierung des lokal etwas vergrößerten Querschnitts der Öffnung 3 ist dabei auch abhängig von der Materialstärke des schlauchförmigen Dichtungsmaterials und vorzugsweise so gewählt, dass im eingepassten Zustand der gewünschte Andruck zwischen Dichtungsmaterial 6 und Wandung der Öffnung gewährleistet ist.

In der schematischen Darstellung de Figur 2 endet das schlauchförmige Dichtungsmaterial 6 vor dem äußeren Rand der Öffnung 3, direkt nach der wulstförmigen Aufweitung 5. In diesem Fall kann das adhäsive Material 7 von der Außenseite her teilweise die Öffnung 3 mit füllen. Diese Anordnung ist besonders vorteilhaft, wenn die Elektrode, wie in Fig. 2, dargestellt aus unterschiedlichen Abschnitten zusammengesetzt ist. Bei einer einfachen Elektrodenleitung mit nur einem Querschnitt kann dagegen das Dichtungsmaterial 6 über den Wulst 5 hinausgeführt werden und die Öffnung 3 beispielsweise auch bis zum äußeren Austritt abdichten.

Die erfindungsgemäße Kontaktdurchführung ist besonders vorteilhaft zu realisieren, wenn sowohl die Elektrode, bzw. die Elektrodenabschnitte, als auch die Öffnung 3 in zylinderförmiger Geometrie vorliegen. In diesem Fall kreisförmiger Querschnitte sind die Andruckverhältnisse zwischen Elektrodenoberfläche, Dichtungsmaterial und Gehäusewandung radialsymmetrisch verteilt. Damit korreliert zwangsläufig eine Zentrierung der Elektrode innerhalb der Öffnung, so dass auch die elektrischen Isolationswerte zur Gehäusewandung der elektronischen Komponente optimiert sind.

Die in der Figur 2 dargestellte Segmentierung der Elektrode ist insbesondere vorteilhaft für die Durchführung eines Kondensatorkontakts, da jeder Elektrodenabschnitt 1, 2 auf die unterschiedlichen Anforderungen im Innen- und Außenraum der elektronischen Komponente zugeschnitten sein kann. Dies betrifft nicht allein die Maße, wie in der Figur als Querschnittsänderung schematisiert, sondern vor allem auch die Wahl des Materials. Für Kondensatorkontakte hat es sich nämlich als vorteilhaft erwiesen, wenn die Elektrode innerhalb eines Kondensatorraums mit dem dort vorhandenen Elektrolyt in Kontakt steht. Dadurch kann in diesem Bereich als gewünschter elektrochemischer Vorgang die Oberfläche der Elektrode (=Anode) bei geeigneter Materialwahl oxidiert werden. Dieser Prozess wird auch als Formierung der Elektrode, bzw. Anodisierung bezeichnet. Besteht die Elektrode beispielsweise aus Aluminium oder einer Aluminium-haltigen Legierung, so bildet sich oberflächlich eine Aluminiumoxidschicht. Es hat sich erwiesen, dass dadurch u. a. das Auftreten von unerwünschten Verlustströmen durch Reformierungsprozesse reduziert werden kann.

Um eine solche Formierung zu ermöglichen, kann als eine Ausführungsform der erfindungsgemäßen Kontaktdurchführung die Oberfläche des zum Kondensator führenden Elektrodenabschnitts 2 so strukturiert werden, dass ein auf der Innenseite der elektronischen Komponente vorhandener Elektrolyt auch im Bereich des Dichtungsmaterials 6 bis an die Elektrodenoberfläche gelangen kann. Dabei kann die Strukturierung unterschiedlich ausgeprägt sein und ist vorzugsweise auf einen definierten Teilbereich des Elektrodenabschnitts 2 beschränkt.

Die Figur 3 zeigt schematisch ein erstes Ausführungsbeispiel für eine Strukturierung der Elektrodenoberfläche im Bereich des Elektrodenabschnitts 2. Dazu weist die Elektrode in diesem Bereich oberflächlich eingeprägte, linienförmig verlaufende Vertiefungen 8 auf. Entlang dieser rinnenförmigen Strukturen kann im zusammengesetzten Zustand der Kontaktdurchführung flüssiger Elektrolyt aus dem Bereich des im Inneren des Komponentengehäuses befindlichen Kondensators zwischen Dichtungsmaterial 6 und Elektrodenoberfläche gelangen und so eine Anodisierung auslösen. Profil, Tiefe, Anzahl, und geometrischer Verlauf der Vertiefungen 8 können dabei in weiten Grenzen variiert werden, so dass die jeweiligen Bedingungen, z. B. die erforderliche Materialmenge an Elektrolyt, für den Formierungsprozess bereitgestellt werden können.

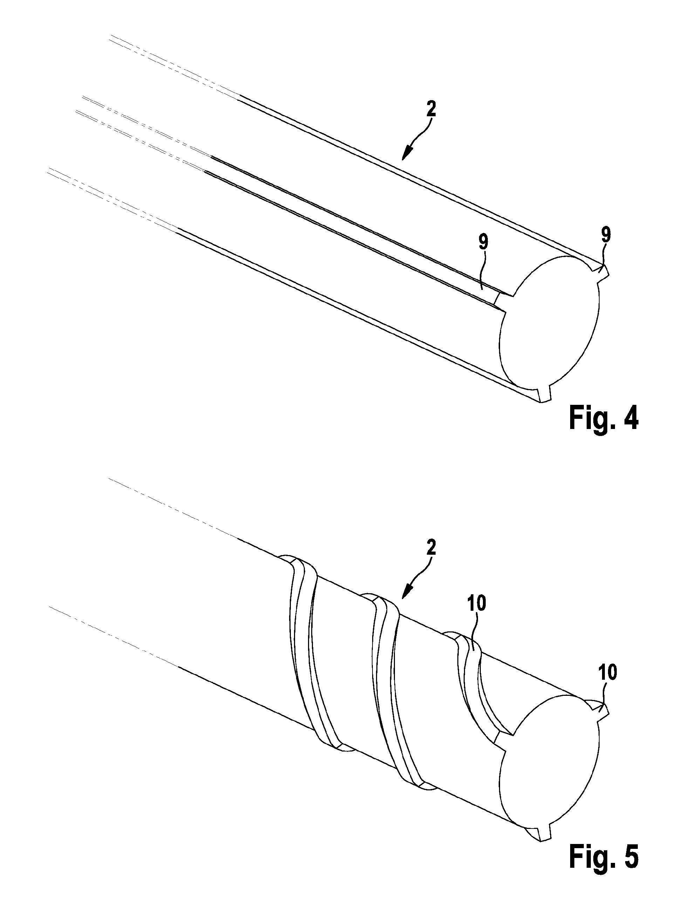

Wie in Figur 4 dargestellt, können anstelle von Vertiefungen in der Elektrodenoberfläche auch Stege 9 oder andere erhabene Prägungen als Strukturierung vorhanden sein. Auch entlang solcher Erhebungen entstehen unter dem Dichtungsmaterial 6 kleine, z.B. kanalförmige Zwischenräume, die dann als Diffusionswege für Elektrolyt dienen.

In Figur 3 und 4 sind die Strukturierungen 8 bzw. 9 mit einem linearen Verlauf parallel zur Elektrodenachse dargestellt. Andere geometrische Anordnungen sind darüber hinaus ebenso möglich. Als ein Beispiel zeigt die Figur 5 einen spiralförmig verlaufenden Steg 10. Ebenso ist es nicht zwingend erforderlich, dass die Strukturen 8, 9 oder 10 durchgängig linienförmig aufgebaut sind, beispielsweise führt auch eine Relief-artige Verteilung von einzelnen Erhebungen (Stegabschnitte, Noppen) oder Mulden zu verbleibenden Zwischenräumen über die Elektrolyt durch Kapillarwirkung zur Elektrodenoberfläche vordringen kann.

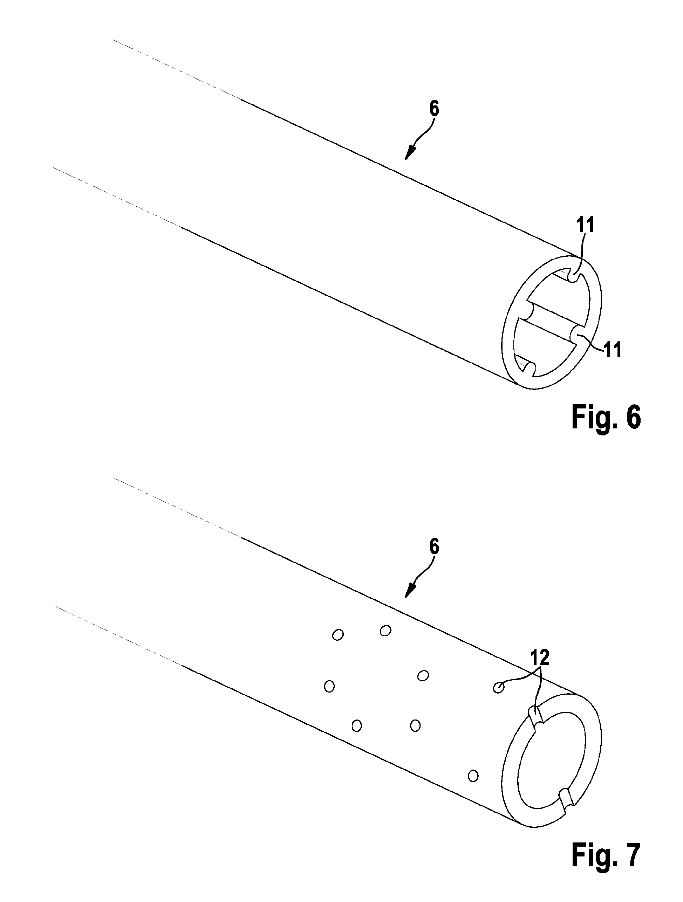

Das für eine Anodisierung notwendige Heranführen von Elektrolyt kann alternativ oder auch zusätzlich zur dargestellten Strukturierung der Elektrodenoberfläche durch entsprechende Ausgestaltung des Dichtungsmaterials erfolgen. Die Figur 6 zeigt als Ausführungsbeispiel schlauchförmiges Dichtungsmaterial 6, bei dem die innere Oberfläche linienförmige Erhebungen 11 aufweist, die bei der zusammengesetzten Kontaktdurchführung als Transportkanäle für Elektrolyt wirken können. Ganz analog wie bei der beschriebenen Strukturierung der Elektrodenoberfläche können ebenso auch rillenförmige Vertiefungen vorgesehen sein (nicht dargestellt) und auch hier sind wieder auch andere Formen (Noppen, Mulden usw.) und unterschiedliche geometrische Anordnungen möglich. Derartige Strukturen lassen sich bereits bei der Fabrikation eines schlauchförmigen elastischen Materials mit bekannten Prägetechniken sehr einfach herstellen.

Die Figur 7 zeigt eine weitere erfindungsgemäße Ausgestaltung des Dichtungsmaterials 6. In diesem Beispiel weist das Dichtungsmaterial 6 eine Verteilung von Poren 12 in dem Bereich auf, in dem eine Reformierung der Elektrode 2 erfolgen soll. Derartige Öffnungen lassen sich mit bekannten Techniken (z. B. LASER-Bohren oder Stanzen) beim Fertigungsprozess des schlauchförmigen Dichtungsmaterials mit gewünschtem Durchmesser und beliebiger Flächendichte herstellen. Vorzugsweise liegt der Durchmesser der Poren im Mikro- bis Millimeterbereich. Dabei sind die Anzahl von Poren pro Flächenelement und der durchschnittliche Porendurchmesser wesentliche Einflussgrößen für die Menge des hindurch tretenden Elektrolyts.

Damit eine Anodisierung des inneren Elektrodenabschnitts 2 auf einen vordefinierten Bereich beschränkt bleibt, genügt es, die Strukturierung der Elektrodenoberfläche und/oder die Strukturierung bzw. Perforierung des Dichtungsmaterials 6 auf den gewünschten Bereich zu beschränken. So können beispielsweise die linienförmigen Vertiefungen 8 nur bis an die wulstförmige Aufweitung 5 der Elektrode 2 herangeführt werden. Damit wirkt im weiteren Verlauf der Wulst 5 zusammen mit dem dort vollständig anliegenden und porenfreien Dichtungsmaterial 6 als zuverlässige Abdichtung und verhindert ein weiteres Vordringen des Elektrolyts.

Alle dargestellten erfindungsgemäßen Ausführungsbeispiele zur Heranführung von Elektrolyt an vordefinierte Bereiche eines Elektrodenabschnitts lassen sich beliebig kombinieren. Damit ist bei der Gestaltung einer Kontaktdurchführung sowohl Menge als auch Verteilung des Elektrolyts in großem Umfang variierbar, so dass in Hinblick auf die Eigenschaften des Elektrodenmaterials die für den Formierungsprozess optimalen Bedingungen vorgegeben werden können.

## Patentansprüche

1. Kontaktdurchführung für eine elektronische Komponente, wobei die Kontaktdurchführung eine Öffnung (3) in der Wandung (4) eines Komponentengehäuses, eine leitfähige Elektrode (1,2) und ein elastisches Dichtungsmaterial aufweist, wobei durch die Öffnung (3) in der Wandung (4) des Komponentengehäuses die leitfähige Elektrode (1, 2) geführt ist, die im Bereich der Öffnung (3) zumindest abschnittsweise von dem elastischen Dichtungsmaterial (6) umgeben ist, welches für Flüssigkeiten undurchlässig ist und isolierende elektrische Eigenschaften aufweist, wobei zumindest ein Abschnitt der Elektrode (2) im Bereich der Durchführung durch die Öffnung (3) einen lokal vergrößerten Querschnitt, vorzugsweise in Form eines wulstförmigen Rundprofils (5) aufweist, so dass in diesem Bereich das zwischen Elektrode und Wandung der Öffnung (3) angeordnete elastische Dichtungsmaterial (6) dergestalt komprimiert wird, dass durch den Andruck des Dichtungsmaterials (6) auf die Elektrodenoberfläche und die Wandung (4) der Öffnung (3) eine für Flüssigkeiten undurchlässige Abdichtung gebildet wird, **dadurch gekennzeichnet, dass** der von der Abdichtung aus ins Innere der elektronischen Komponente führende Abschnitt der Elektrode (2) als Kondensatorkontakt ausgeführt ist und
- in diesem Bereich vom Dichtungsmaterial (6) schlauchförmig umgeben ist, wobei dieser Abschnitt der Elektrode (2) eine Oberflächenstruktur in Form von Vertiefungen (8) und/oder Erhebungen (9, 10) dergestalt aufweist, dass zwischen Dichtungsmaterial (6) und Elektrodenoberfläche Kapillaren verbleiben, über die Flüssigkeiten zur Elektrodenoberfläche diffundieren kann, oder
- vom Dichtungsmaterial (6) schlauchförmig umgeben ist und wobei das Dichtungsmaterial (6) nur in diesem Bereich zur Elektrodeseite hin eine O berflächenstruktur in Form von Vertiefungen (8) und/oder Erhebungen (9, 10) dergestalt aufweist, dass zwischen Dichtungsmaterial (6) und Elektro denoberfläche Kapillaren verbleiben, über die Flüssigkeiten zur Elektroden Oberfläche diffundieren kann.

2. Kontaktdurchführung nach Anspruch 1, **dadurch gekennzeichnet, dass** die elektronische Komponente ein Kondensator und die Flüssigkeit ein Elektrolyt ist.

3. Kontaktdurchführung nach Anspruch 1, **dadurch gekennzeichnet, dass** die elektronische Komponente ein Kondensator für elektrische Implantate ist.

4. Kontaktdurchführung nach einem der vorangehenden Ansprüche, **dadurch gekennzeichnet, dass** die Öffnung (3) in der Wandung (4) des Komponentengehäuses vorzugsweise im zentralen Bereich des Durchgangs lokal einen vergrößerten Querschnitt mit gleichem oder ähnlichen Profil des lokal vergrößerten Querschnitts der Elektrode aufweist, wobei der Abschnitt der Elektrode (2) mit vergrößertem Querschnitt (5) zusammen mit dem umgebenden komprimierten Dichtungsmaterial (6) formschlüssig in diesen Bereich der Öffnung (3) eingesetzt ist.

5. Kontaktdurchführung nach einem der vorangehenden Ansprüche, **dadurch gekennzeichnet, dass** die Körperseitige Öffnung (3) und die dort austretende Elektrode (1) mittels adhäsivem Material (7), z. B. Epoxydharz versiegelt ist.

6. Kontaktdurchführung nach einem der vorangehenden Ansprüche, **dadurch gekennzeichnet, dass** die Vertiefungen (8) und/oder Erhebungen (9, 10) auf der Oberfläche des Elektrodenabschnitts (2) linienförmig, z.B. geradlinig oder spiralförmig ausgebildet sind.

7. Kontaktdurchführung nach einem der vorangehenden Ansprüche, **dadurch gekennzeichnet, dass** die Vertiefungen (8) und/oder Erhebungen (9, 10) auf der Oberfläche des schlauchförmigen Dichtungsmaterials (6) linienförmig, z. B. geradlinig oder spiralförmig ausgebildet sind.

8. Kontaktdurchführung nach einem der vorangehenden Ansprüche, wobei der von der Abdichtung aus ins Innere der elektronischen Komponente führende Abschnitt der Elektrode (2) als Kondensatorkontakt ausgeführt ist und vom Dichtungsmaterial (6) schlauchförmig umgeben ist, wobei das Dichtungsmaterial (6) nur in diesem Bereich eine Verteilung von Poren (11) aufweist, über die Flüssigkeiten zur Elektrodenoberfläche diffundieren kann.

9. Kontaktdurchführung nach einem der vorangehenden Ansprüche , **dadurch gekennzeichnet, dass** der von der Abdichtung aus ins Innere der elektronischen Komponente führende Abschnitt der Elektrode (2) aus Aluminium oder einer Aluminiumlegierung besteht.

10. Kontaktdurchführung nach einem der vorangehenden Ansprüche , **dadurch gekennzeichnet, dass** der von der Abdichtung aus ins Innere der elektronischen Komponente führende Abschnitt der Elektrode (2) an der Oberfläche eine durch Formierungsprozess mit dem Elektrolyt gebildete Oxidschicht aufweist.

11. Kontaktdurchführung nach einem der vorangehenden Ansprüche, **dadurch gekennzeichnet, dass** der von der Abdichtung aus ins Innere der elektronischen Komponente führende Abschnitt der Elektrode (2) aus einem anderen Metall bzw. einer anderen Metalllegierung besteht als der von der Abdichtung aus zur Körperseite verlaufende Elektrodenabschnitt (1).

12. Elektrisches Implantat beinhaltend eine elektronische Komponente mit einer Kontaktdurchführung nach einem der vorangehenden Ansprüche.

## Claims

1. A feedthrough contact for an electronic component, wherein the feedthrough contact has an opening (3) in the wall (4) of a component housing, a conductive electrode (1, 2), and an elastic sealing material, wherein the conductive electrode (1, 2) is routed through the opening (3) in the wall (4) of the component housing and is surrounded at least in portions in the region of the opening (3) by the elastic sealing material (6), which is impermeable to fluids and has insulating electrical properties,
wherein
at least one portion of the electrode (2) has a locally enlarged cross section, preferably in the form of a bulge-shaped round profile (5), in the region of the feedthrough through the opening (3), such that the elastic sealing material (6) disposed between the electrode and the wall of the opening (3) is compressed in this region such that the pressing of the sealing material (6) against the electrode surface and the wall (4) of the opening (3) creates a seal that is impermeable to fluids,
**characterised in that**
the portion of the electrode (2) extending from the seal into the interior of the electronic component is formed as a capacitor contact and
- is surrounded in this region by sealing material (6), wherein this portion of the electrode (2) has a surface structure in the form of recesses (8) and/or raised areas (9, 10), in such a way that capillaries remain between the sealing material (6) and electrode surface, via which fluids can diffuse to the electrode surface, or
- is surrounded in a tubular manner by the sealing material (6), and wherein the sealing material (6) only in this region towards the electrode side has a surface structure in the form of recesses (8) and/or raised areas (9, 10), in such a way that capillaries remain between the sealing material (6) and electrode surface, via which fluids can diffuse to the electrode surface.

2. The feedthrough contact according to Claim 1, **characterised in that** the electronic component is a capacitor and the fluid is an electrolyte.

3. The feedthrough contact according to Claim 1, **characterised in that** the electronic component is a capacitor for electrical implants.

4. The feedthrough contact according to one of the preceding claims, **characterised in that** the opening (3) in the wall (4) of the component housing, preferably in the central region of the passage, has a locally enlarged cross section, the profile of which is identical or similar to that of the locally enlarged cross section of the electrode, wherein the portion of the electrode (2) having the enlarged cross section (5) is inserted together with the surrounding, compressed sealing material (6), into this region of the opening (3) in a form-fitting manner.

5. The feedthrough contact according to one of the preceding claims, **characterised in that** the body-side opening (3) and electrode (1) emerging therefrom are sealed using adhesive material (7), for example epoxy resin.

6. The feedthrough contact according to one of the preceding claims, **characterised in that** the recesses (8) and/or raised areas (9, 10) are formed on the surface of the electrode portion (2) as lines, for example as straight lines or spiralled lines.

7. The feedthrough contact according to one of the preceding claims, **characterised in that** the recesses (8) and/or raised areas (9, 10) are formed on the surface of the tubular sealing material (6) as lines, for example as straight lines or spiralled lines.

8. The feedthrough contact according to one of the preceding claims, wherein the portion of the electrode (2) extending from the seal into the interior of the electronic component is designed as a capacitor contact and is surrounded in a tubular manner by the sealing material (6), wherein the sealing material (6) only in this region has a distribution of pores (11), via which the fluids can diffuse to the electrode surface.

9. The feedthrough contact according to one of the preceding claims, **characterised in that** the portion of the electrode (2) extending from the seal into the interior of the electronic component consists of aluminium or an aluminium alloy.

10. The feedthrough contact according to one of the preceding claims, **characterised in that** the portion of the electrode (2) extending from the seal into the interior of the electronic component has an oxide layer formed on the surface by a forming process with the electrolyte.

11. The feedthrough contact according to one of the preceding claims, **characterised in that** the portion of the electrode (2) extending from the seal into the interior of the electronic component consists of a different metal or a different metal alloy than the electrode portion (1) extending from the seal towards the body side.

12. An electrical implant containing an electronic component having a feedthrough contact according to one of the preceding claims.

## Revendications

1. Passage de contact pour un composant électronique, dans lequel le passage de contact présente un orifice (3) dans la paroi (4) d'un boitier de composant, une électrode (1, 2) conductrice et un matériau d'étanchéité élastique, dans lequel l'électrode conductrice (1, 2) est menée à travers l'orifice (3) dans la paroi (4) du boitier de composant, électrode qui est au moins partiellement entourée dans la zone de l'orifice (3) par le matériau d'étanchéité (6) élastique, lequel est imperméable vis-à-vis des liquides et présente des propriétés électriques isolantes,
où
au moins une portion de l'électrode (2) présente une section transversale agrandie localement, de préférence sous la forme d'un profilé rond (5) en forme de renflement dans la zone du passage, de sorte que, dans cette zone, le matériau d'étanchéité (6) élastique disposé entre l'électrode et la paroi de l'orifice (3) est comprimé de telle façon qu'une étanchéité imperméable vis-à-vis des liquides est créée par la pression du matériau d'étanchéité (6) sur la surface de l'électrode et la paroi (4) de l'orifice (3),
**caractérisé en ce**
**que** la portion de l'électrode (2) menant à partir de l'étanchéité vers l'intérieur du composant électronique est conçue sous la forme d'un contact de condensateur et
- est entouré comme par un tuyau par le matériau d'étanchéité (6) dans cette zone, où cette portion de l'électrode (2) présente une structure de surface sous forme de creux (8) et/ou d'élévations (9, 10) de telle façon que des capillaires restent conservés entre le matériau d'étanchéité (6) et la surface d'électrode, par lesquels du liquide peut diffuser vers la surface d'électrode, ou
- est entouré comme par un tuyau par le matériau d'étanchéité (6) et dans lequel le matériau d'étanchéité présente uniquement dans cette zone vers le côté de l'électrode une structure de surface sous la forme de creux (8) et/ou d'élévations (9, 10) de telle façon que des capillaires restent conservés entre le matériau d'étanchéité (6) et la surface d'électrode, par lesquels du liquide peut diffuser vers la surface d'électrode.

2. Passage de contact selon la revendication 1, **caractérisé en ce que** le composant électronique est un condensateur et que le liquide est un électrolyte.

3. Passage de contact selon la revendication 1, **caractérisé en ce que** le composant électronique est un condensateur pour des implants électriques.

4. Passage de contact selon l'une des revendications précédentes, **caractérisé en ce que** l'ouverture (3) dans la paroi (4) du boîtier de composant présente de préférence une section transversale agrandie localement avec un profil identique ou analogue à la section transversale agrandie localement de l'électrode dans la zone centrale du passage, dans lequel la portion de l'électrode (2) avec la section transversale (5) agrandie est insérée, ensemble, par complémentarité des formes, avec le matériau d'étanchéité (6) comprimé l'entourant, dans cette zone de l'orifice (3).

5. Passage de contact selon l'une des revendications précédentes, **caractérisé en ce que** l'ouverture (3) du côté du corps et l'électrode (1) en ressortant à cet endroit est scellée au moyen d'un matériau (7) adhésif, par exemple, une résine époxyde.

6. Passage de contact selon l'une des revendications précédentes, **caractérisé en ce que** les creux (8) et/ou les élévations (9, 10) à la surface de la portion d'électrode (2) sont conçues sous forme de lignes, par exemple, de lignes droites ou sous forme de spirales.

7. Passage de contact selon l'une des revendications précédentes, **caractérisé en ce que** les creux (8) et/ou les élévations (9, 10) à la surface du matériau d'étanchéité (6) en forme de tuyau sont conçus sous forme de lignes, par exemple, de lignes droites ou sous forme de spirales.

8. Passage de contact selon l'une des revendications précédentes, dans lequel la portion d'électrode (2) s'étendant à partir de l'étanchéité vers l'intérieur du composant électronique est conçue sous la forme d'un contact de condensateur et est entourée par la matériau d'étanchéité (6) en forme de tuyau, dans lequel le matériau d'étanchéité (6) présente une distribution de pores (11) uniquement dans cette zone, par l'intermédiaire desquels les liquides peuvent diffuser à la surface d'électrode.

9. Passage de contact selon l'une des revendications précédentes, **caractérisé en ce que** la portion d'électrode (2) s'étendant à partir de l'étanchéité vers l'intérieur du composant électronique est constituée d'aluminium ou d'un alliage d'aluminium.

10. Passage de contact selon l'une des revendications précédentes, **caractérisé en ce que** la portion d'électrode (2) s'étendant à partir de l'étanchéité vers l'intérieur du composant électronique présente à la surface une couche d'oxyde formée avec l'électrolyte par un processus de transformation.

11. Passage de contact selon l'une des revendications précédentes, **caractérisé en ce que** la portion d'électrode (2) s'étendant à partir de l'étanchéité vers l'intérieur du composant électronique est constituée d'un autre métal, respectivement, d'un autre alliage métallique, que la portion d'électrode (1) s'étendant à partir de l'étanchéité vers le côté du corps.

12. Implant électrique contenant un composant électronique avec un passage de contact selon l'une des revendications précédentes.
